# FASCICULE DE BREVET EUROPEEN

(11) **EP 1 221 957 B1**
(45) Date de publication et mention de la délivrance du brevet: **31.05.2006**
(21) Numéro de dépôt: 00967952.3
(22) Date de dépôt: 05.10.2000
(51) Int. Cl.: A61K 35/56, A61K 8/98, A61P 17/00, A61P 29/00

(54) **PROCEDE DE PREPARATION D'UNE COMPOSITION PAR EXTRACTION DE NACRE ET SON UTILISATION EN COSMETIQUE ET DERMATOLOGIE**
VERFAHREN ZUR HERSTELLUNG EINER ZUSAMMENSETZUNG DURCH EXTRAKTION VON PERLMUTTER UND SEINE KOSMETISCHE UND DERMATOLOGISCHE VERWENDUNG
METHOD FOR PREPARING A COMPOSITION BY MOTHER-OF-PEARL EXTRACTION,AND USE THEREOF IN COSMETICS AND DERMATOLOGY

(30) Priorité: 05.10.1999 FR 9912409
(43) Date de publication de la demande: 17.07.2002
(73) Titulaire: CENTRE NATIONAL DE LA RECHERCHE SCIENTIFIQUE (CNRS), 75794 Paris Cedex 16 (FR)
(72) Inventeur: LOPEZ, Evelyne, F-75007 Paris (FR); CHEMOUNI, Alfred, Edouard, F-75019 Paris (FR)
(74) Mandataire: Warcoin, Jacques
(86) Numéro de dépôt international: PCT/FR2000/002766
(87) Numéro de publication internationale: WO 2001/024804

(56) Documents cités:
- WO-A-97/24133

## Description

La présente invention se rapporte à un procédé de préparation d'une composition comprenant l'intégralité des composants de la nacre, au moins un collagène et au moins un protéoglycanne, à la composition susceptible d'être obtenue par ce procédé et à l'utilisation de cette dernière tant dans les domaines pharmaceutique que cosmétique, en particulier pour lutter contre les effets du vieillissement cutané et/ou des phanères.

Les mammifères se protègent du milieu extérieur par une barrière constituée par la peau, tissu très structuré, composé de plusieurs couches, mais sensible aux agressions dues aux variations du milieu axtracorporel. Cette situation est unique dans le règne animal puisque les poissons et les grenouilles sécrètent du mucus, les oiseaux sont couverts de plumes et les vertébrés submammaliens et mammaliens, sauf les hominidés actuels, sont couverts de poils.

L'être humain a perdu ces attributs protecteurs : il a une peau qu'il faut protéger et qu'il est possible de stimuler afin de l'aider à lutter contre les agressions et le vieillissement.

En particulier, on sait que le vieillissement est un phénomène physiologique qui se traduit notamment par un amincissement de la peau et une perte de l'élasticité, conduisant notamment à l'apparition de rides plus ou moins profonde. On peut également constater un relâchement ou dessèchement superficiel et une pigmentation anarchique.

La peau comprend trois couches : l'épiderme, le derme et, en profondeur, l'hypoderme.

L'enveloppe protectrice la plus externe de la peau, l'épiderme, qui recouvre étroitement le derme, est constituée en surface du stratum corneum. Le stratum corneum est fait de deux couches : le stratum disjunctum en surface dont la particularité est la desquamation et le stratum compactum, le plus profond, qui joue un rôle de barrière. L'épiderme, facile à observer macroscopiquement puisque superficiel, a fait l'objet de nombreuses études. Les réponses connues sont celles entraînées par certains agents du milieu extracorporel ou bien celles résultant d'applications de substances actives d'origines diverses. Les cellules de l'épiderme résultent de l'activité des cellules de la couche basale qui reposent sur une membrane basale séparant l'épiderme du derme.

Le derme résulte de l'activité biosynthétique des fibroblastes, qui élaborent les constituants de la matrice extracellulaire. Celle-ci est formée de quatre grandes familles de macromolécules : les collagènes, l'élastine, les glycoprotéines de structure et les protéoglycannes. Le derme a la faculté de répondre aux signaux donnés par l'épiderme qui en réponse envoie également des signaux à l'épiderme. D'une manière générale, il existe des échanges entre ces différentes couches dermiques et épidermiques de la peau, qui sont destinés à assurer le renouvellement cellulaire, la cohésion et l'hydratation des couches externes.

De nombreux actifs ont été proposés pour prévenir ou retarder les effets du vieillissement.

Parmi ceux-ci, la nacre a été utilisée depuis l'Antiquité en esthétique et dans les pharmacopées traditionnelles. Par ailleurs, la nacre est connue pour ses propriétés régénératrices des os.

On sait en particulier que la nacre, ou aragonite chonchylifère, est une formation minéralisée biogène; elle est constituée d'une matrice organique de substances fibreuses et non fibreuses représentant environ 1,7 à 2 % de la masse totale (Taylor et al, Bulletin of the British Museum (Natural history) Zoology.Suppl.3125pp. + 29 Planches, 1969) et de carbonate de calcium cristallisé sous forme orthorhombique, appelé aragonite, associé à des oligo-éléments (sodium, magnésium, lanthane, zinc, brome, césium, fer, manganèse, chlore, cuivre, potassium, calcium, strontium et soufre). Plus particulièrement, la phase organique totale de la nacre se présente sous la forme d'une matrice organique composée de protéines fibreuses, constituées notamment de collagènes ancestraux dépourvus d'hydroxyproline et d'hydroxylysine, et de protéines non fibreuses. Environ 50% de la matrice organique de la nacre est hydrosoluble (ou "water soluble"). Les 50 % restants ne peuvent être obtenus qu'après décalcification.

Toutefois, les produits de l'art antérieur, obtenus par mélange d'une poudre de nacre avec un excipient ou un diluant inerte, pulvérulent, comme décrit par exemple dans la demande WO 97/23231, provoquent des phénomènes indésirables d'irritation de la peau, dus la présence de la nacre sous forme de poudre (en particulier de son composant majoritaire, l'aragonite ou CaCO₃). La teneur en poudre de nacre dans ces produits doit donc impérativement être très limitée pour éviter ces phénomènes d'irritation. Ces pénomènes indésirables d'irritation ont notamment pour origine le pH trop basique de ces produits connus, au contact de la peau. En outre, les produits connus à base de poudre de nacre présentent des problèmes spécifiques de formulations, notamment de stabilité (ruptures d'émulsions) et d'ajustement du pH à une valeur moins basique.

Par ailleurs, la demande WO 97/24133 décrit un procédé de préparation de substances biologiquement actives à partir de la nacre, par mise en contact d'une poudre de nacre avec un solvant aqueux choisi parmi l'eau pure, bidistillée ou apyrogène, éventuellement additionné de sels, dans lequel on sépare ensuite la fraction hydrosoluble de façon à ne récupérer que la fraction aqueuse, essentiellement donc dépourvue des constituants minéraux et de la phase organique non hydrosoluble de la nacre. Ce procédé ne peut donc permettre en particulier la préparation d'une composition comprenant notamment l'intégralité des composants de la nacre, en particulier la phase organique totale de la nacre.

On a maintenant constaté de manière tout à fait surprenante et inattendue que la mise en oeuvre d'un procédé spécifique permet d'obtenir une composition nouvelle, comprenant non seulement l'intégralité des composants de la nacre mais aussi une association avantageuse de ces derniers avec certains composés qui permettent au moins de potentialiser l'activité des composants de la nacre, sinon de fournir un effet de synergie, en vue notamment de prévenir et / ou de réduire de manière visible les effets liés au vieillissement de la peau et/ou des phanères.

La présente invention a ainsi pour objet un procédé de préparation d'une composition, caractérisé en ce qu'il comprend les étapes consistant :
a) à réduire de la nacre en une poudre de granulométrie comprise entre environ 1 et environ 300 µm;
b) à mettre la poudre de nacre ainsi obtenue en contact intime avec un agent d'extraction sous la forme d'une solution hydroglycolique d'au moins un collagène, d'au moins un protéoglycanne ou d'un mélange de ces derniers; puis
c) à récupérer le mélange d'extraction, formé par la mise en contact intime, constituant la composition désirée.

Le présent procédé permet d'obtenir une composition sous la forme du mélange d'extraction obtenu à l'étape b).

La nacre utilisée pour la mise en oeuvre du procédé selon l'invention peut être obtenue à partir de coquilles de mollusques nacriers et de certains céphalopodes (par exemple le nautile). En particulier, on l'obtient à partir d'huîtres telles que *Pinctada maxima.*

On utilise la nacre brute, débarassée des autres éléments de coquille riches en polysaccharides et calcite. De préférence, on part de nacre blanche, sinon il faut prévoir une étape d'élimination des pigments qui peuvent provoquer des intolérances.

Il s'agit d'une matière première aisément disponible, dont l'utilisation n'a pas d'impact écologique négatif sur les populations naturelles. En effet, la plupart de ces huîtres ou autres mollusques nacriers font l'objet d'une aquaculture.

De plus, un avantage supplémentaire est donné par le fait que la matière première peut être obtenue à partir de coquilles d'huîtres ayant produit des perles ; en effet, une huître perlière est éliminée du pool productif après avoir produit successivement au maximum 3 perles, alors qu'elle possède une épaisse couche de nacre de qualité excellente (grade A). La présente invention propose donc un débouché supplémentaire pour une utilisation de la nacre en aval de la perliculture.

La granulométrie de la poudre de nacre utilisée pour la mise en oeuvre du présent procédé est comprise entre environ 1 et environ 300 µm, telle que mesurée avec des moyens classiques à la portée de l'homme du métier, comme la technique de tamisage et/ou la technique à lecture LASER.

Selon un mode de mise en oeuvre particulier du procédé selon l'invention, la nacre est réduite en une poudre de granulométrie comprise entre environ 50 et environ 100 µm.

Selon un autre mode de mise en oeuvre particulier, la nacre est réduite en une poudre de granulométrie comprise entre environ 15 et environ 50 µm, ce qui permet d'améliorer le rendement, en approchant de la taille de l'unité cristalline (par exemple, l'unité élémentaire de la nacre de *Pinctada maxima* est le biocristal, un cristal hexagonal d'aragonite très volumineux de 9 à 12 µm).

On procède en particulier de la manière suivante pour réduire la nacre en poudre selon l'étape a) du présent procédé.

Dans une première phase, la nacre brute est débarrassée de la partie externe de la coquille (périostracum) par meulage ou tout autre procédé non dénaturant.

Les plaques de nacre sont ensuite réduites en fragments par concassage afin de pouvoir ensuite procéder à une micronisation. Les fragments destinés à être micronisés ont de préférence une longueur comprise entre environ 2 à environ 5 centimètres et une épaisseur d'environ 0,3 centimètres.

Dans une seconde phase, ces fragments sont micronisés.

La nacre peut être utilisée sans décontamination préalable. Dans le cas d'une décontamination préalable, on réalise un lavage décontaminant très rapide, sans trempage des fragments, dans une solution d'hypochlorite de sodium à 6,6% de chlore actif (12°). Le séchage doit être fait extemporanément de façon à retirer toute trace d'eau.

Le meulage doit être réalisé à sec dans des jarres en zirconium réservées exclusivement à cet effet, précédemment lavées (eau javellisée, rinçage, puis lavage à l'eau distillée) et stérilisées à chaud. Le concassage se fait au moyen de billes en zirconium, elles-mêmes stérilisées.

La stérilisation de la nacre brute réduite en poudre peut être réalisée de deux façons différentes :
- stérilisation par irradiation aux rayons γ 2,5 Mrad;
- stérilisation à chaud pendant 1 à 2 heures dans un autoclave à 100 °C. La nacre n'est dégradée (matrice minérale et matrice organique) qu'à partir d'environ 250 °C (Balmain, Hannoyer and Lopez, "Fourier transform infrared spectroscopy (FTIR) and X-ray diffraction analyses of mineral and organic matrix during heating of Mother of Pearl (nacre) from the shell of the mollusc Pinctada Maxima", J. Biomed. Mater. Res. (Appl. Biomater.), vol.48(5):749-754, 1999). Cette stérilisation ne détruit donc pas ses différents composants.

L'étape b) du procédé selon l'invention consiste à mettre la poudre de nacre décrite ci-dessus en contact intime avec un agent d'extraction sous la forme d'une solution hydroglycolique d'au moins un collagène, d'au moins un protéoglycanne ou d'un mélange de ces derniers.

Par "solution hydroglycolique", on entend selon l'invention une solution du collagène et du protéoglycanne obtenue en utilisant un solvant hydroglycolique c'est à dire, d'une manière générale, un solvant sous forme d'un mélange d'eau et d'au moins un glycol.

Par "glycol", on entend de manière connue tout composé possédant deux fois la fonction alcool. En particulier, le glycol pouvant être utilisé est choisi dans le groupe constitué par l'éthylène glycol, le propylène glycol, le butylène glycol et les mélanges de ces derniers.

L'eau pouvant être utilisée pour former le solvant hydroglycolique peut être de l'eau pure, bidistillée, apyrogène, déminéralisée ou encore de l'eau déionisée.

Le rapport pondéral eau:glycol dans le solvant hydroglycolique est de préférence compris entre environ 1 : 100 et environ 100 : 1 , et plus particulièrement entre environ 1 : 1 et environ 20 : 1 .

De préférence, l'agent d'extraction est une solution hydroglycolique d'au moins un collagène.

Le collagène pouvant être utilisé en solution hydroglycolique dans l'agent d'extraction selon l'invention peut être tout collagène constituant la substance intercellulaire du tissu conjonctif, disponible dans le règne animal, connu de l'homme du métier.

On utilise tout particulièrement de préférence un collagène marin, c'est à dire un collagène issu d'un organisme d'origine marine tels que les vertébrés marins. En particulier, le collagène marin est le principal constituant des tissus conjonctifs du poisson, où il tient un rôle essentiel dans la structure de la peau, des muscles, des tendons et des ligaments.

On peut citer tout particulièrement le collagène marin "PANCOGENE^{R} MARIN", tel que commercialisé par la société Gattefossé (Saint Priest, France), dont la dénomination INCI est "Soluble Collagen" (référencement au Japon : "water soluble collagen"; MHV : 20800CZY0010000). Il s'agit d'un collagène extrait de peaux de poissons d'espèces non protégées des mers chaudes appartenant à la classe des Téléostéens.

On peut également citer le collagène marin "COLLAGENE NATIF MARIN - Code 690", tel que commercialisé par la société Laboratoire Industriel de Biologie (Soisy Sous Montmorency, France) dont la dénomination CTFA est "Amino Collagen Amino Acid". Il s'agit d'un collagène acide soluble dans l'eau, extrait des peaux de poissons.

De préférence, on utilise une concentration en collagène comprise entre environ 0,0001 et environ 50 % en poids, et plus particulièrement entre environ 0,01 et environ 15 % en poids, par rapport au poids total de l'agent d'extraction.

L'agent d'extraction est également de préférence une solution hydroglycolique d'au moins un protéoglycanne.

Le protéoglycanne pouvant être utilisé en solution hydroglycolique dans l'agent d'extraction selon l'invention peut être tout protéoglycanne connu de l'homme du métier, notamment tout protéoglycanne non soufré.

En particulier, le protéoglycanne est choisi dans le groupe constitué par l'acide hyaluronique, le condroitine sulfate, le dermatane sulfate, l'éparane sulfate, le kératane sulfate et les mélanges de ces derniers.

L'acide hyaluronique est tout particulièrement préféré, tel que celui commercialisé par la société Laboratoire Industriel de Biologie (Soisy Sous Montmorency, France).

La concentration en protéoglycanne utilisé est de préférence comprise entre environ 0,0001 et environ 40 % en poids, et plus particulièrement entre environ 0,01 et environ 10 % en poids, par rapport au poids total de l'agent d'extraction.

Bien entendu, l'agent d'extraction peut comprendre en outre tout composé additionnel connu de l'homme du métier, approprié pour l'étape d'extraction elle même ou en vue des utilisations possibles de la composition préparée par le présent procédé, comme celles décrites ci-après. L'agent d'extraction peut ainsi par exemple comprendre en outre des agents complexants tels que l'EDTA (acide (éthylène-diamine)tétraacétique).

Selon un mode de réalisation particulièrement préféré de la présente invention, on met la poudre de nacre en contact intime avec l'agent d'extraction selon l'étape b) en réalisant un mélange, constitué par la poudre de nacre et l'agent d'extraction, tel qu'il comprend, par rapport à son poids total, environ 20 à environ 60 % en poids de poudre de nacre obtenue à l'étape a), telle que décrite ci-dessus, et le reste en agent d'extraction tel que décrit ci-dessus.

La mise en contact intime de la poudre de nacre avec l'agent d'extraction, selon l'étape b) du présent procédé, peut notamment être effectuée par mise en suspension de la poudre de nacre dans l'agent d'extraction, sous agitation mécanique forte et homogène, pour permettre un enrobage d'extraction des particules de nacre.

On peut par exemple procéder à température ambiante c'est à dire à une température de l'ordre de 20 °C pendant environ 1 heure, mais les durées et les températures peuvent être adaptées, par l'homme du métier, notamment en fonction de la granulométrie de départ de la poudre de nacre.

Selon un autre mode de réalisation, l'étape b) de mise en contact peut être effectuée par passage sous pression de l'agent d'extraction à travers la poudre de nacre immobilisée. Cette immobilisation peut par exemple être effectuée par une colonne, de type CLHP, éventuellement en mélange avec des substances de charge permettant un meilleure diffusion de l'agent d'extraction et évitant le compactage de la poudre de nacre.

Sans vouloir toutefois être lié à une quelconque théorie, on pense que la présence du collagène et du protéoglycanne dans l'agent d'extraction, en particulier du collagène marin et de l'acide hyaluronique, favoriserait l'extraction de composés apparentés présents dans la nacre, par un phénomène physico-chimique d'affinité, avec en outre une potentialisation des effets des composants ainsi extraits de la nacre. En particulier, on pense que l'extraction des collagènes ancestraux de la nacre serait favorisée par la présence des collagènes marins et l'extraction tant des protéines d'adhésion (décorine et cytokines) que des protéoglycannes de la nacre serait favorisée par la présence du protéoglycanne choisi.

Quoi qu'il en soit, de préférence, on réalise la mise en contact intime, pour une température donnée, pendant un temps suffisant pour réaliser une extraction pratiquement complète. Par "extraction pratiquement complète", on entend selon l'invention l'extraction de tous les composants extractibles de la nacre lors de sa mise en contact avec l'agent d'extraction.

En d'autres termes, l'obtention d'un équilibre des concentrations totales notamment en collagènes ou en protéoglycannes dans la phase liquide hydroglycolique correspond à l'obtention de ladite "extraction pratiquement complète".

Il est donc à la portée de l'homme du métier de déterminer le caractère "pratiquement complet" d'une telle extraction, par exemple par des mesures régulières de ces concentrations totales en collagènes ou en protéoglycannes dans la phase liquide hydroglycolique.

Quoi qu'il en soit , l'étape de mise en contact peut comprendre avant sa fin une période de repos (arrêt de l'agitation) de la suspension de la poudre de nacre dans l'agent d'extraction.

A la fin de l'étape b) de mise en contact, on récupère le mélange d'extraction, formé par la mise en contact intime, constituant la composition désirée.

Cette composition, sous forme d'une suspension hydroglycolique, peut être conservée telle qu'elle avant utilisation. Pour des raisons pratiques de mise en oeuvre ultérieure (stockage, transport, formulation etc.) on peut également séparer la phase liquide de la phase solide de cette composition.

Ainsi, selon une variante, le procédé selon l'invention est caractérisé en ce que, à la fin de l'étape b), on récupère le mélange d'extraction, formé par la mise en contact intime, constituant la composition désirée, et l'on sépare la phase liquide de la phase solide de la composition par des moyens connus de l'homme du métier, tels que des moyens d'ultra-filtration, de filtration tangentielle etc.

On comprend que les phases solide et liquide ainsi séparées constituent ensemble la composition préparée comme décrit ci-dessus. On peut ainsi prévoir que, lors d'une formulation ultérieure utilisant cette composition, avec des excipients pharmaceutiquement ou cosmétiquement appropriés, la composition peut être reconstituée à toute étape de la formulation, par adjonction simultanée ou séparée dans le temps des phases solide et liquide respectives, pour des raisons de commodités de mise en formulation.

La présente invention a également pour objet une composition nouvelle, sucsceptible d'être obtenue par le procédé tel que décrit ci-dessus (comprenant les phases solides et liquides, avec ou sans étape de séparation comme décrit ci-dessus).

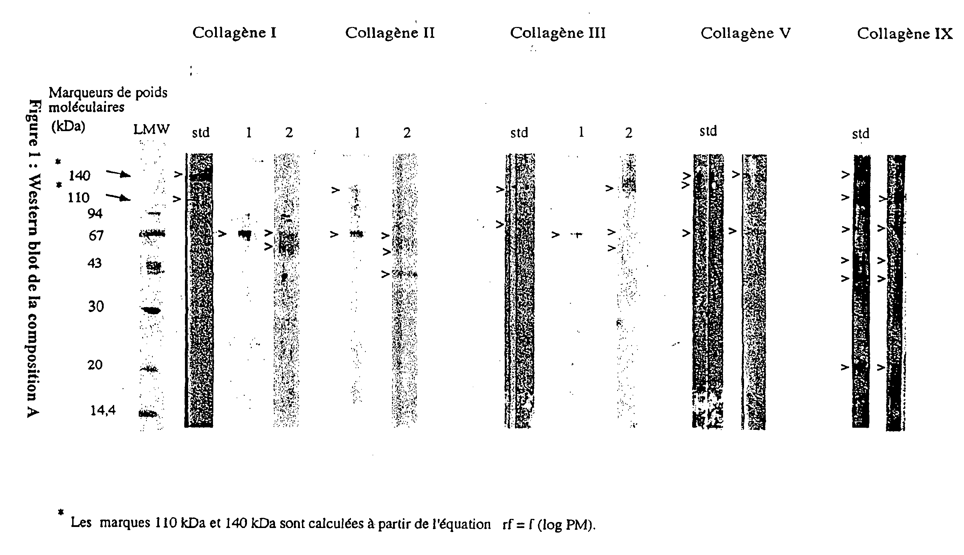

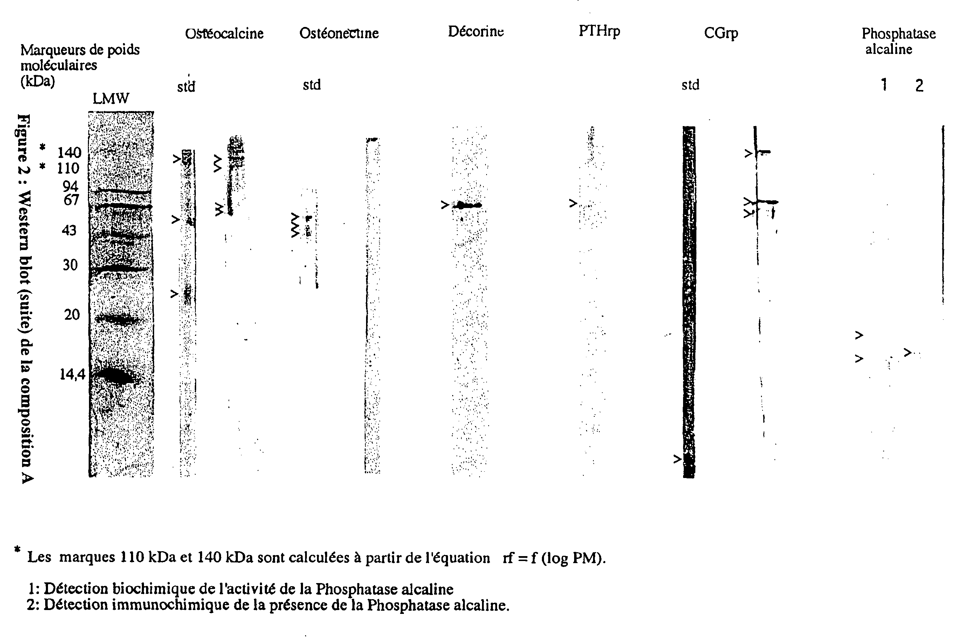

En particulier, cette composition est caractérisée par le fait qu'elle comprend au moins, sous forme d'une suspension hydroglycolique d'une phase liquide et d'une phase solide :
- de l'aragonite (CaCO₃) ;
- des oligo-éléments choisis dans le groupe constitué par le sodium, le magnésium, le lanthane, le zinc, le brome, le césium, le fer, le manganèse, le chlore, le cuivre, le potassium, le calcium, le strontium, le soufre et les mélanges de ces derniers;
- des protéines fibreuses de la nacre, notamment des collagènes ancestraux de la nacre apparentés aux collagènes marins de l'agent d'extraction (voir Figure 1 décrite ci-après);
- des protéines non fibreuses de la nacre, notamment des protéines apparentées aux protéines d'adhésion telles que la Décorine (voir Figure 2 décrite ci-après);
- au moins un collagène non issu de la nacre et/ou au moins un protéoglycanne non issu de la nacre.

On comprend bien entendu que par "collagène non issu de la nacre" et par "protéoglycanne non issu de la nacre" on entend respectivement selon l'invention le collagène et le protéoglycanne utilisés pour l'agent d'extraction de l'étape b) du présent procédé. De même, l'expression "suspension hydroglycolique" s'explique par le fait que la composition selon l'invention comprend non seulement des composants solubles (en solution hydroglycolique) mais aussi des composants non solubles (notamment l'aragonite).

Par ailleurs, les préférences énoncées ci-dessus pour le procédé selon l'invention s'appliquent bien entendu à cette composition.

Ainsi, en particulier, le collagène non issu de la nacre dans la composition selon l'invention est de préférence un collagène marin et plus particulièrement un collagène marin choisi dans le groupe constitué par le "PANCOGENE^{R} MARIN", le "COLLAGENE NATIF MARIN - Code 690" et les mélanges de ces derniers. De même, le protéoglycanne non issu de la nacre peut être tout protéoglycanne connu de l'homme du métier, notamment tout protéoglycanne non soufré, et est en particulier choisi dans le groupe constitué par l'acide hyaluronique, le condroitine sulfate, le dermatane sulfate, l'éparane sulfate, le kératane sulfate et les mélanges de ces derniers, et est plus particulièrement l'acide hyaluronique.

Comme le montrent plus en détail les exemples ci-après, cette composition présente des propriétés très intéressantes vis à vis de la peau et/ou des phanères, notamment des propriétés de régénération tissulaire, permettant par exemple une cicatrisation améliorée, ainsi que des propriétés anti-âge permettant de prévenir et / ou de réduire de manière visible les effets liés au vieillissement de la peau et/ou des phanères. Au niveau des différents types cellulaires de la peau, elle présente une action réparatrice et régulatrice de l'équilibre physiologique entre ses différents constituants. Plus particulièrement, ses composantes organiques et minérales agissant en particulier à plusieurs niveaux sur le métabolisme des kératinocytes. Cette composition permet une restructuration de l'épiderme, contribuant à une meilleure protection des couches les plus profondes. L'épiderme devient plus résistant, plus profond et en continuelle interaction avec le derme. De plus, cette composition permet un enrichissement en élastine et en collagènes. La peau est plus élastique et plus ferme. Elle est résistante et ses composantes se renouvellent à un rythme soutenu. En outre, cette composition stabilise les synthèses pigmentaires et favorise la microcirculation. Enfin, cette composition présente l'avantage d'une innocuité totale et celui d'avoir un effet anti-inflammatoire, donc apaisant.

Ainsi, la présente invention a également pour objet une composition pharmaceutique, caractérisée en ce qu'elle comprend, en tant que principe actif, la composition nouvelle telle que décrite ci-dessus, susceptible d'être obtenue par le procédé selon l'invention, et un excipient pharmaceutiquement acceptable.

De préférence, l'excipient pharmaceutiquement acceptable est un excipient approprié pour une application dermatologique.

La présente invention a également pour objet l'utilisation de cette composition nouvelle telle que décrite ci-dessus, susceptible d'être obtenue par le procédé tel que décrit ci-dessus, pour la fabrication d'un médicament destiné au traitement des troubles de la régénération tissulaire de la peau et/ou des phanères.

Par ailleurs, la présente invention a pour objet l'utilisation de la composition nouvelle telle que décrite ci-dessus, susceptible d'être obtenue par le procédé tel que décrit ci-dessus, pour la fabrication d'un médicament destiné au traitement des troubles de la peau et/ou des phanères liés au vieillissement.

Enfin, la présente invention a pour objet l'utilisation de la composition nouvelle telle que décrite ci-dessus, susceptible d'être obtenue par le procédé tel que décrit ci-dessus, pour la fabrication d'un médicament destiné au traitement des manifestations cutanées inflammatoires.

La présente invention a encore pour objet une composition cosmétique, caractérisée en ce qu'elle comprend, en tant que principe cosmétiquement actif, la composition nouvelle telle que décrite ci-dessus, susceptible d'être obtenue par le procédé selon l'invention, et un excipient cosmétiquement acceptable.

On peut utiliser tout excipient cosmétique connu de l'homme du métier. Bien entendu, la composition cosmétique selon l'invention peut contenir en outre des ingrédients de type cosmétique connus de l'homme du métier, tels que des agents hydratants, des agents adoucissants, ou encore des filtres chimiques ou organiques.

Les compositions pharmaceutiques et cosmétiques décrites ci-dessus peuvent en particulier se présenter sous la forme d'une crème, d'une pommade, d'un gel, d'une lotion, d'une émulsion huile-dans-eau, d'une émulsion eau-dans-huile ou peuvent être associées à tout vecteur pharmaceutiquement ou cosmétiquement acceptable tels que des liposomes.

En outre, la présente invention a pour objet l'utilisation de la composition nouvelle telle que décrite ci-dessus, susceptible d'être obtenue par le procédé selon l'invention, pour la fabrication d'une composition cosmétique destinée au traitement cosmétique pour la régénération tissulaire de la peau et/ou des phanères.

Par ailleurs, la présente invention a pour objet l'utilisation de la composition nouvelle telle que décrite ci-dessus, susceptible d'être obtenue par le procédé selon l'invention, pour la fabrication d'une composition cosmétique destinée au traitement cosmétique des modifications liées au vieillissement de la peau et/ou des phanères.

La présente invention a enfin pour objet une méthode de traitement cosmétique pour la régénération tissulaire de la peau et/ou des phanères, caractérisée en ce qu'on applique sur la peau et/ou les phanères la composition nouvelle telle que décrite ci-dessus, suceptible d'être obtenue par le procédé selon l'invention.

La présente invention a par ailleurs pour objet une méthode de traitement cosmétique des modifications liées au vieillissement de la peau et/ou des phanères, caractérisée en ce qu'on applique sur la peau et/ou les phanères la composition nouvelle telle que décrite ci-dessus, suceptible d'être obtenue par le procédé selon l'invention.
Les Figures 1 et 2 représentent un Western blot d'une composition préparée par le procédé selon l'invention.
La Figure 3 représente un histogramme de la composition globale en acides aminés de la phase protéique d'une composition préparée par le procédé selon l'invention.
Les Figures 4 et 5 sont des photographies illustrant l'effet de la composition préparée selon l'invention sur la synthèse de cytokératines par des kératinocytes humains.
Les Figures 6 et 7 sont des photographies illustrant l'effet de la composition préparée selon l'invention sur des kératinocytes humains en culture sevrés en oestradiol.
Les Figures 8 et 9 sont des photographies illustrant l'effet de la composition préparée selon l'invention sur la synthèse de cytokératines par des kératinocytes humains en culture sevrés en oestradiol.
Les Figures 10 et 11 sont des photographies illustrant l'effet de la composition préparée selon l'invention sur la synthèse d'élastine par les fibroblastes humains.
Les Figures 12 et 13 sont des photographies illustrant l'effet des oestrogènes sur des fibroblastes humains.
Les Figures 14 et 15 sont des photographies illustrant l'effet de la composition préparée selon l'invention sur des fibroblastes humains sevrés en oestradiol.
La figure 16 est un histogramme illustrant l'effet de la composition préparée selon l'invention sur les mélanocytes par la modification de la quantité de mélanine captée par des kératinocytes mis en culture en présence de la composition.
Les Figures 17 et 18 sont des photographies illustrant l'effet de la composition préparée selon l'invention sur la quantité de mélanine captée par des kératinocytes humains cultivés.
La Figure 19 est un histogramme illustrant l'effet de la composition préparée selon l'invention sur la sécrétion de l'interleukine 1 (IL-1) par les cellules promyélocytiques HL 60.
Enfin, la Figure 20 est un histogramme illustrant l'étude de la non cytotoxicité de la composition préparée selon l'invention.

Les exemples suivants sont destinés à illustrer la présente invention mais ne doivent en aucun cas être interprétés comme pouvant en restreindre la portée.

### Exemple 1 : procédé de préparation d'une composition selon l'invention

Le matériel utilisé doit être un matériel type sous vide, bien étanche, de préférence en Inox. Il doit être précédemment lavé et rincé proprement de façon à éviter tout risque de contaminations bactériennes.

**1.1)** On utilise de la nacre de *Pinctada maxima* déjà débarrassée de la partie externe de la coquille (périostracum) et mise sous forme de fragments irréguliers (fournie par la société Pharma Futura 10, rue de Charonne, 75012 PARIS). Les fragments sont de taille comprise entre 2 à 5 centimètres de longueur et 0,3 centimètres d'épaisseur.

On effectue un lavage décontaminant très rapide sans trempage des fragments dans une solution d'hypochlorite de sodium à 6,6% de chlore actif (12°) puis un séchage extemporanément de façon à retirer toute trace d'eau.

Le meulage pour la micronisation doit être réalisé à sec dans des jarres en zirconium réservées exclusivement à cet effet, précédemment lavées (eau javellisée + rinçage + lavage eau distillée) et stérilisées à chaud. Le broyage se fait au moyen de billes en zirconium, elles-mêmes stérilisées.

La poudre de nacre micronisée ainsi obtenue présente une granulométrie comprise entre 50 et 150 µm. Elle est ensuite stérilisée par irradiation aux rayons γ 2,5 Mrad.

La poudre de nacre micronisée ainsi réalisée présente les caractéristiques suivantes :
- Aspect Pulvérulent
- Couleur Blanche
- Odeur Caractéristique de la Nacre
- Arsenic < 1ppm
- Métaux lourds < 10 ppm.
Analyse bactériologique :
- Numéro des germes aérobies 1 550/g
- Degré d'hygrométrie 0,3 %

**1.2)** On réalise le mélange suivant : 400 grammes de poudre de nacre de Pinctada Maxima micronisée comme décrit ci-dessus (granulométrie comprise entre 50 et 150 µm) sont ajoutés progressivement à 600 ml d'un agent d'extraction sous forme d'une solution hydroglycolique (eau et éthylèneglycol respectivement selon un rapport pondéral 10 : 1) comprenant 0,18 % en poids du collagène marin "PANCOGENE^{R} MARIN" (commercialisé par la société Gattefossé; Saint Priest, France; dénomination INCI : "Soluble Collagen"; référencement au Japon : "water soluble collagen"; MHV : 20800CZY0010000), et 0,2 % en poids d'acide hyaluronique (commercialisé par la société Laboratoire Industriel de Biologie; Soisy Sous Montmorency, France), par rapport au poids total de l'agent d'extraction, sous agitation à environ 4000 tours/minutes et à une température d'environ 20°C. L'addition étant terminé, on maintient l'agitation et la température pendant 60 minutes. L'agitation doit être forte et homogène de façon à mettre toutes les particules du mélange en contact et permettre un enrobage d'extraction.

Le mélange est ensuite laissé au repos pendant 6 heures.

La composition ainsi obtenue se compose d'une phase solide particulaire blanc-cassé, en suspension dans une phase liquide et a un pH d'environ 8.

Cette composition comprend :
- de l'aragonite (CaCO3);
- des oligo-éléments marins : sodium, magnésium, lanthane, zinc, brome, césium, fer, manganèse, chlore, cuivre, potassium, calcium, strontium et soufre (voir Tableau 1 ci-dessous);
- des protéines fibreuses : différents types de protéines apparentées aux collagènes marins, pouvant être considérées comme des collagènes ancestraux de la nacre, et le collagène marin "PANCOGENE^{R} MARIN";
- des protéines non fibreuses : protéoglycannes, dont l'acide hyaluronique de la nacre et l'agent d'extraction, ainsi que des protéines d'adhésion de la nacre (décorine rp et cytokines rp; "rp signifiant "related peptides or proteins" ou "peptides ou protéines apparentés", c'est à dire des peptides ou protéines apparentés respectivement à la décorine et à la cytokine dans la mesures où ils sont reconnus par les anticorps tournés contre ces dernières molécules).

Les techniques utilisées pour l'identification des peptides et protéines dans le cadre de la présente invention sont celles décrites dans les articles suivants :
- électrophorèse - Méthode SDS-Page : méthode de Laenli, Nature 1970, 227 680-682;
- immunotransfert : Western blot selon Towbin H. et al, 1979, Proc. Natl. Acad. Sci. USA 76(9) : 4350-4354 et Burnette W.N. 1981, Analytical Biochemistry 112 : 195-203.

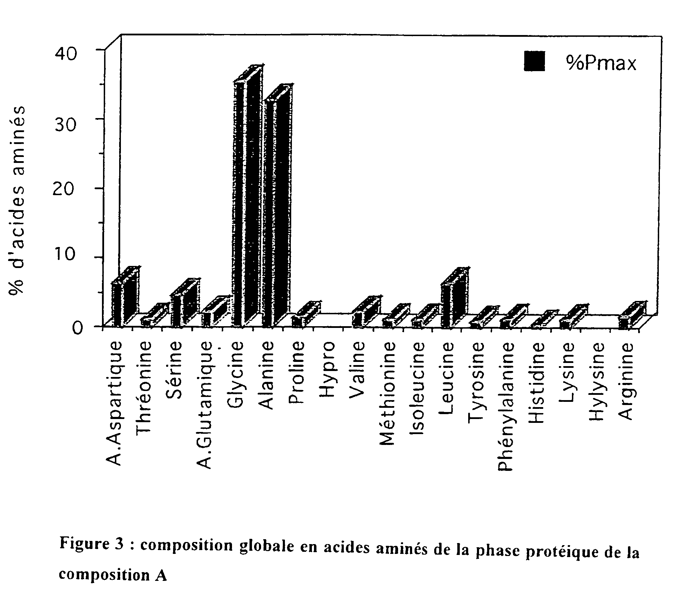

Les Western blot correspondants sont représentés aux Figures 1 et 2. L'analyse en acides aminés de la phase protéique indique la présence d'acide aspartique, de thréonine, de serine, d'acide glutamique, de glycine, d'alanine, de proline, de valine, de methionine, d'isoleucine, de leucine, de tyrosine, de phenylanine, d'histidine, de lysine, et d'arginine. La Figure 3 représente un histogramme de la composition globale en acides aminés.

La composition obtenue comme décrit ci-dessus sera dénommé dans ce qui suit par "composition A".

**Tableau 1 : analyse quantitative des oligo-éléments contenus dans la composition A**

| **Elément** | **Concentration moyenne (µg/g)** | **Elément** | **Concentration moyenne (g/100g)** |
|---|---|---|---|
| Soufre | 0,02 | Calcium | 38,8 |
| Magnésium | 2 | | |
| Lanthane | 0,28 | | |
| Zinc | 0,4 | | |
| Brome | 1,78 | | |
| Césium | 5,5 | | |
| Fer | 13,6 | | |
| Manganèse | 50 | | |
| Chlore | 296 | | |
| Cuivre | 143 | | |
| Potassium | 582 | | |
| Strontium | 1000 | | |
| Sodium | 5420 | | |

### Exemple 2 : étude de l'activité de la composition A

La recherche scientifique, histologique et cytologique qui utilise soit des modèles *in vivo* animaux, soit des cultures de cellules humaines en faisant appel à la microscopie et aux techniques les plus modernes de détection de substances par immunologie et immunocytochimie permet actuellement une étude à l'échelle cellulaire qui a été mise en pratique pour expérimenter les substances actives de cette composition A.

Cette étude a essentiellement porté sur trois types de cellules dans la mesure où celles-ci jouent de manière connue un rôle majeur dans la structure, le fonctionnement et la pigmentation du tissu cutané : les kératinocytes, les fibroblastes et les mélanocytes. Les deux types cellulaires, kératinocyes et fibroblastes, possèdent les récepteurs spécifiques aux substances actives de la composition A et en particulier de l'ensemble des composants de la nacre qui y sont présents, entraînant une cascade de messages pouvant atteindre le niveau des couches les plus profondes du derme.

L'étude a non seulement porté sur leur aspect morphologique mais aussi le comportement de leurs composants majeurs, noyaux et cytoplasme, témoignant de leur activité de synthèse et de leur capacité de renouvellement. La présence de certaines substances actives ainsi que celle de protéines de structure sécrétées a également été identifiée.

### 1.1) Activité de la composition A sur les kératinocytes : Synthèse de cytokératines par des kératinocytes humains cultivés en présence de la composition A

### 1.1.1) Méthode

Les kératinocytes sont les cellules les plus externes de notre corps. Ils constituent notre première barrière de protection. Les kératinocytes interviennent en agissant de manière passive et active. C'est en constituant une barrière qui joue le rôle de bouclier que les kératinocytes élaborent la protection passive. Ce bouclier est fabriqué à partir de cellules desséchées et anuclées. La forte cohésion des cellules permet d'assurer un système très homogène. Les kératinocytes synthétisent des substances, les cytokératines, qu'ils conservent dans leur cytoplasme pour structurer cette barrière. Les cytokératines constituent le squelette internes des kératinocytes. Ce squelette interne donne à ces cellules leur volume et augmente leur capacité de communication intercellulaire et facilite la captation de la mélanine. Plus l'épiderme sera riche en cytokératines, plus il sera efficace et donnera à la peau son aspect jeune. En facilitant le contact entre les cellules, les cytokératines préviennent la perte des NMS (Natural Moisturizing Factors : facteurs d'hydratation naturels, tels que les céramides, le cholestérol et les acides gras).

La protection active de la peau est liée au fait que les kératinocytes sont les premières cellules de notre corps à être en contact avec l'extérieur. Elles ont donc développé une panoplie de composants destinés à informer notre organisme aux éventuels changements des conditions de notre environnement. Pour ce faire, ces cellules sont capables de synthétiser des facteurs de croissance destinés à stimuler les cellules sous jascentes du derme (fibroblastes), notamment le PTH related peptide (PTHrp), synthétisé majoritairement par les kératinocytes et connu pour être un facteur de diférenciation cellulaire.

La méthode utilisée a été la suivante. Elle met en oeuvre une technique semi-quantitative rapide qui permet d'apprécier le taux de chacune des protéines sécrétées présentes dans le cytoplasme cellulaire.

4.10⁴ cellules sont ensemencées par chambres de 0,9 cm² (lames de 8 chambres NUNC) et mises en culture pendant une nuit. Après rinçage avec du tampon HBSS, le milieu à 1% de la composition A ou le milieu de contrôle (à 0% de composition A) est ajouté (450 µl par chambre).

Les cultures sont incubées 48 h. Après élimination des surnageants et rinçage des cultures, les cellules sont fixées avec du paraformaldéhyde pendant 30 min à 4°C puis rincées avec du tampon PBS.

200 µl d'anticorps primaires anti-cytokératines sont ajoutés. L'incubation dure 30 min à température ambiante. Les surnageants sont alors éliminés et les cellules sont rincées avec le PBS.

200 µl d'anticorps secondaires reconnaissant l'anticorps primaire et couplés à un marqueur fluorescent, en l'occurence la fluorescéine, sont ajoutés. Après 30 min d'incubation à température ambiante, les surnageants sont éliminés et les cellules sont rincées au PBS. Les lames sont alors montées puis examinées au microscope inversé à fluorescence (détection par immunofluorescence de cytokératines intracytoplasmiques).

La quantité de protéines synthétisées, en l'occurence des cytokératines, par les cellules et ainsi marquées est proportionnelle à l'intensité de la fluorescence résultant du marquage immunologique.

### 1.1.2) Résultats

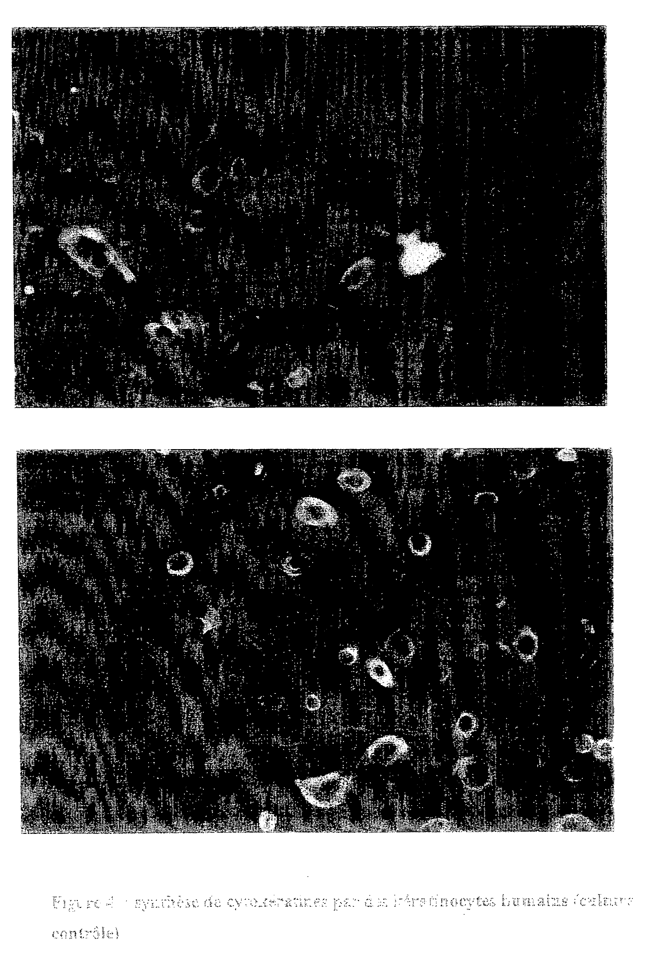

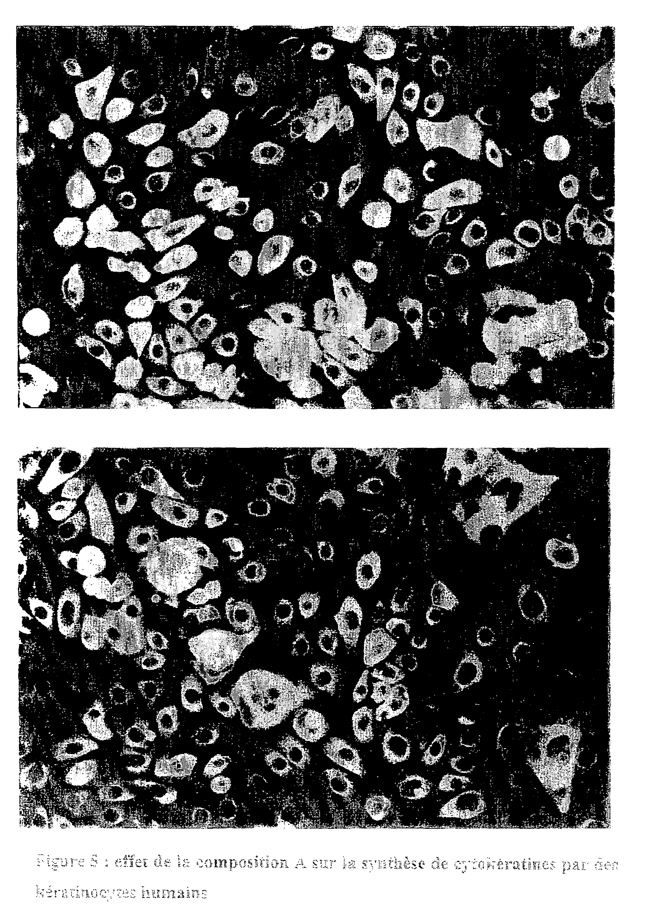

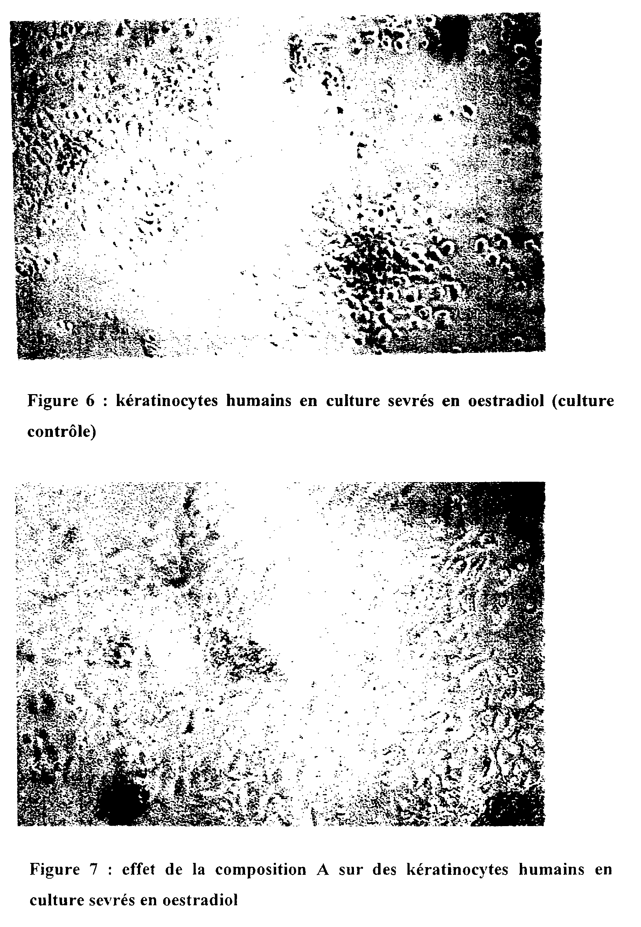

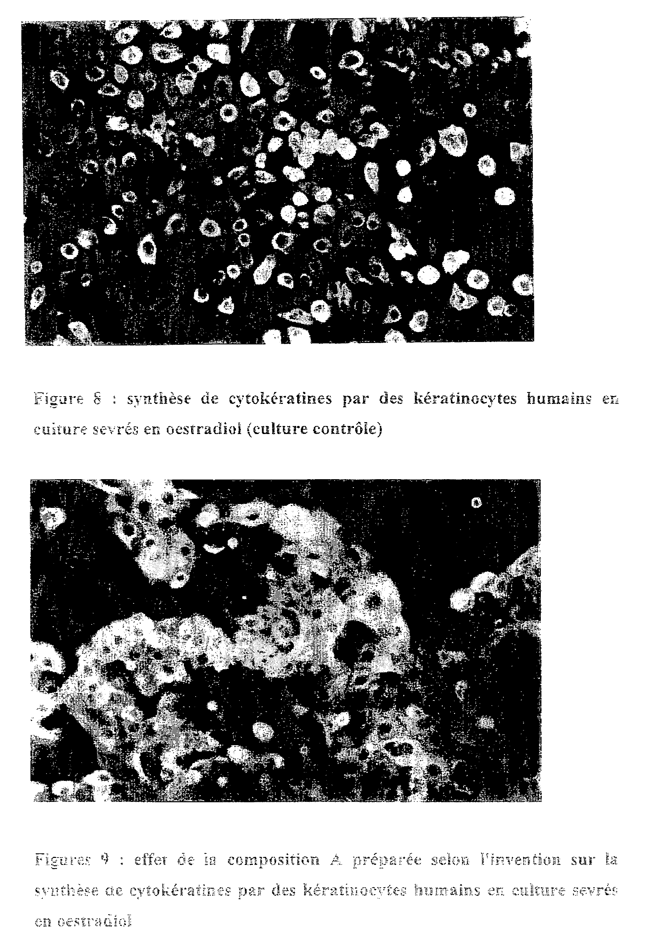

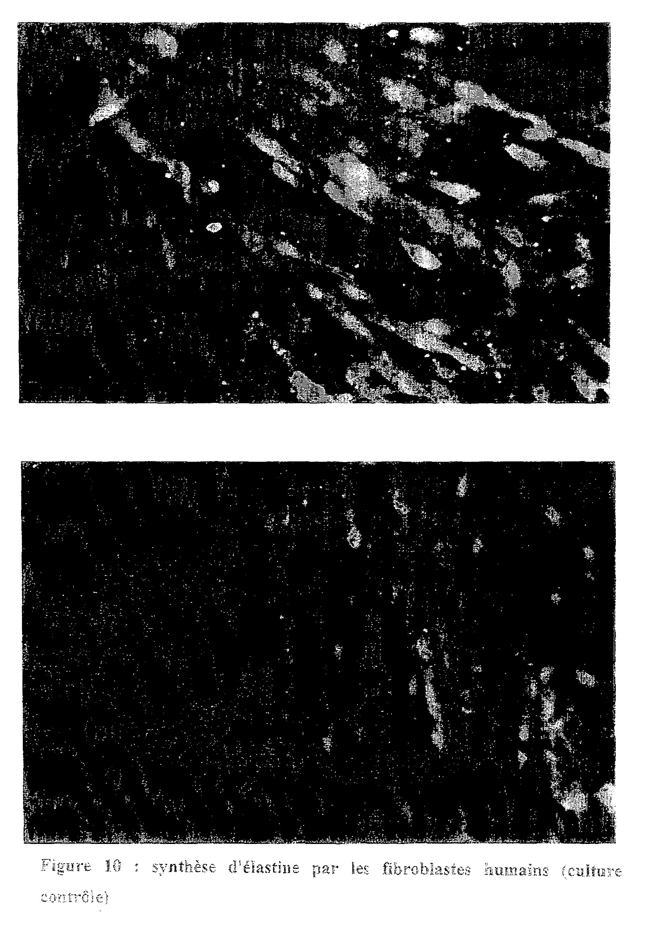

Comme le montre les Figures 4 et 5, l'addition de 0,5% de la composition A dans le milieu de culture des kératinocytes a pour effet d'augmenter la synthèse des cytokératines. La composition A restaure l'activité des kératinocytes provenant de peaux âgées ainsi que la sécrétion de cytokératines par ces kératinocytes (action sur des kératinocytes sevrés en oestradiol; voir Figures 6, 7 et 8, 9).

En outre, la synthèse par les kératinocytes de PTHrp calcium dépendant est stimulée en présence de la composition A. Le PTHrp, synthétisé majoritairement par les kératinocytes, est particulièrement actif en présence de l'ion calcium fourni par la composition A et agit en synergie avec les cytokines de la matrice organique totale de la nacre présente dans la composition A.

La décorine, protéine d'adhésion majeure de la régénération cutanée est également stimulée en présence de la composition A.

Les cadhérines, protéines d'adhésion calcium dépendantes, sont aussi grandement responsables de la cohésion. Elles sont stimulées en présence de la composition A.

### 1.2) Activité de la composition A sur les fibroblastes, synthèse d'élastine

### 1.2.1) Synthèse d'élastine par des fibroblastes humains cultivés en présence de la composition A

La méthode utilisée est la même que celle décrite au paragraphe 1.1.1) ci-dessus pour l'étude sur les kératinocytes.

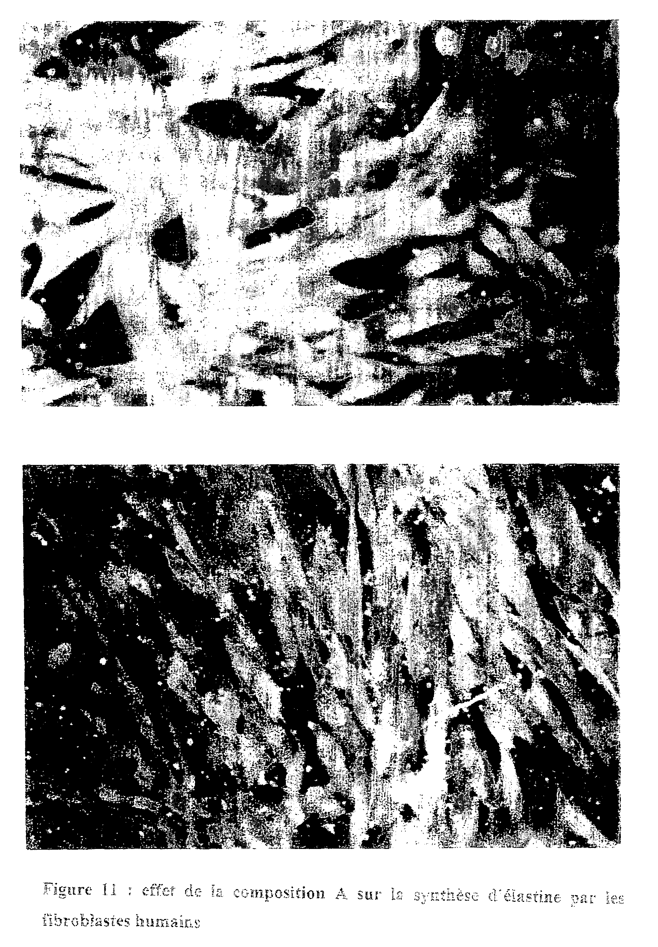

Comme cela ressort des Figures 10 et 11, la composition A à 1% de concentration stimule fortement la synthèse d'élastine par les fibroblastes humains.

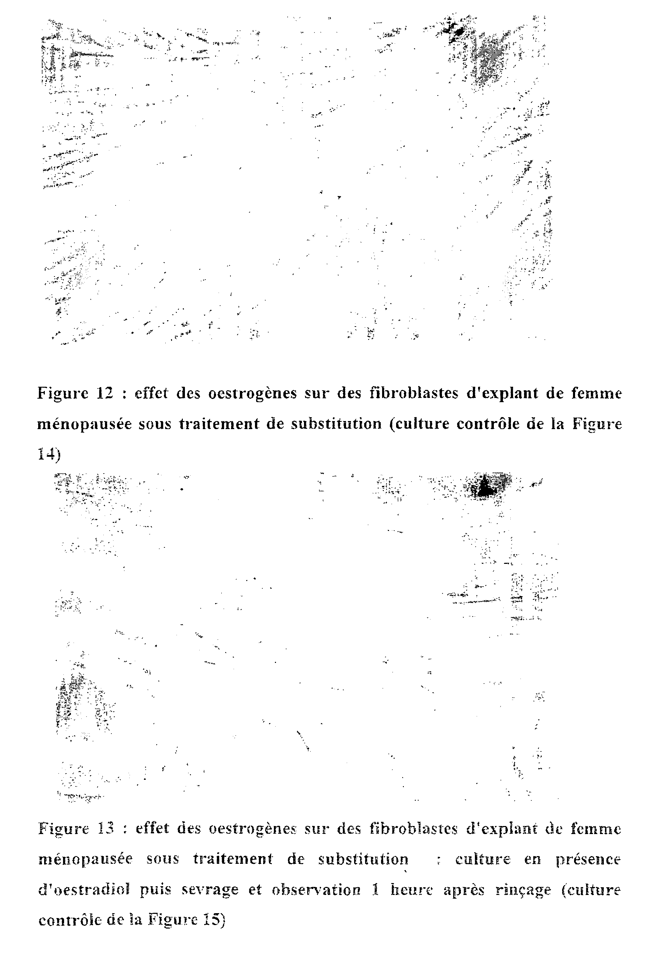

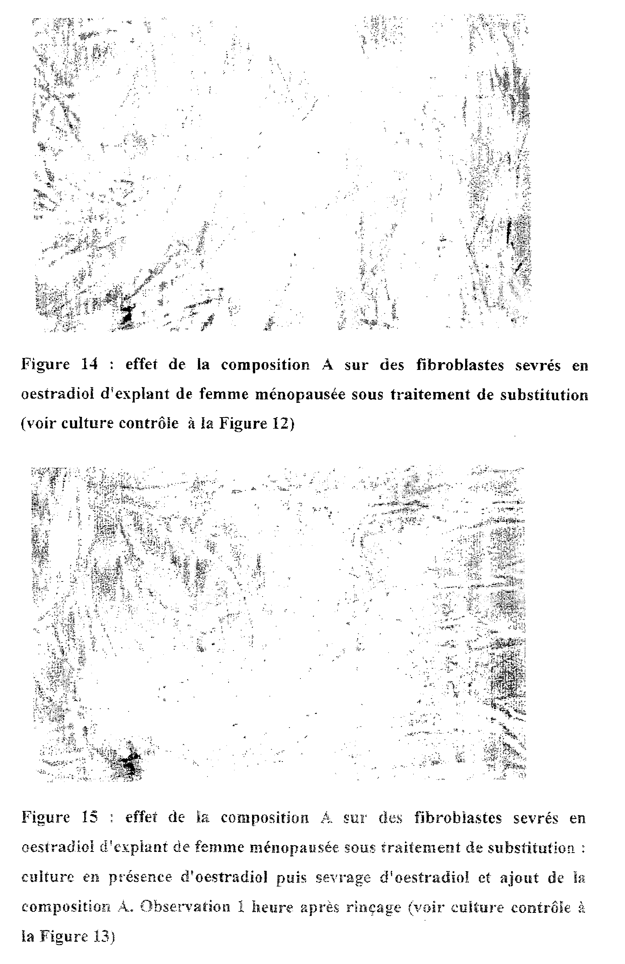

### 1.2.2) Comparaison de l'effet des oestrogènes et du Biocrystal maxima m. sur des fibroblastes humains en culture (Figures 12, 13, 14 et 15).

Les fibroblastes matures peu actifs sont de forme très allongée, aplatie et présentent des noyaux denses. Les fibroblastes actifs ont des noyaux de grande taille, arrondis, avec de volumineux nucléoles, témoignant du processus actif de synthèse. Le type "fibroblaste actif est observé dans les cultures d'explants de peau prélevés sur une femme de 50 ans sous traitement hormonal de substitution.

La privation d'oestrogènes dans le milieu de culture provoque une inactivation drastique des cellules. Elles ne sont plus adhérentes ni confluentes, certaines présentent un début de pycnose.

Les Figures 12 et 14 illustrent l'effet de la composition A sur des fibroblastes humains provenant d'explants de femme de 50 ans ménopausée (donc en baisse de sécrétion d'oestrogènes), sous traitement de substitution.

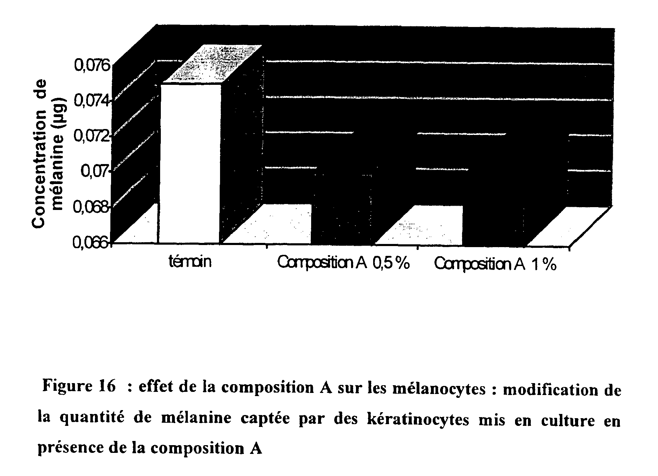

Les Figures 13 et 15 illustrent l'effet de la composition A sur des fibroblastes humains sevrés en oestradiol, provenant d'explants de femme de 50 ans ménopausée, sous traitement de substitution.

Comme cela ressort de ces Figures 12 à 15, la composition A, ajoutée à 1% au milieu de culture restaure complètement l'activité des fibroblastes sevrés. L'action de la composition A est remarquable : les fibroblastes ont un noyau bien développé, très clair, avec un nucléole proéminent. Leurs corps cellulaires fusiformes s'orientent et sont confluents, permettant les échanges cellulaires. Des résultats similaires ont été obtenus en travaillant sur des cellules provenant d'explants de peaux jeunes.

### 1.3) Activité de la composition A sur les mélanocytes : modification de la quantité de mélanine captée par des kératinocytes mis en culture en présence de la composition A.

### 1.3.1) Méthode

Les mélanocytes sont les cellules pigmentaires responsables de la synthèse de la mélanine, pigment qui est à l'origine de la couleur de notre peau. Les cellules mélanocytaires représentent environ 2 à 4 % de la population épidermique totale. La couleur homogène de la peau est due à une répartition du pigment sur toute la surface cutanée grâce au transfert de la mélanine sous forme de mélanosomes depuis les cellules sécrétrices (mélanocytes) vers les kératinocytes voisins qui la captent.

La méthode consiste à réaliser une co-culture kératinocytes/mélanocytes avec dosage de la mélanine.

Les kératinocytes et les mélanocytes sont toutes deux des populations cellulaires situées au niveau de l'épiderme. Elles sont obtenues à partir d'un prélèvement de peau après élimination du derme et digestion enzymatique de l'épiderme. Les cellules épidermiques ainsi isolées sont comptées au Coulter Counter (Coultronics) puis elles sont mises en culture dans un milieu de culture classique type Dubelco modifié (eagle medium) supplémenté avec de la glutamine, strptomycine/péniciline et 10% de serum de veau foetal. Les pourcentages sont ajustés en fonction des résultats obtenus. Ce milieu permet la survie des mélanocytes et des kératinocytes.

Après 4 jours d'incubation en présence de la composition A, les cellules sont digérées par un mélange NaOH/DMSO puis le surnageant est récupéré après centrifugation. La lecture est effectuée à 470 nm.

### 1.3.2) Résultats

Les résultats sont regroupés dans le tableau 1 suivant et représentés par l'histogramme à la Figure 16.

**Tableau 2 : µg de mélanine captée par les kératinocytes**

| | **échantillon 1** | **échantillon 2** | **échantillon 3** | **moyenne** | **écart type** |
|---|---|---|---|---|---|
| témoin | 0,150 | 0,149 | 0,148 | 0,149 | 0,001 |
| Composition A (0,5 %) | 0,150 | 0,132 | 0,140 | 0,141 | 0,009 |
| Composition A(1%) | 0,139 | 0,137 | 0,143 | 0,140 | 0,003 |

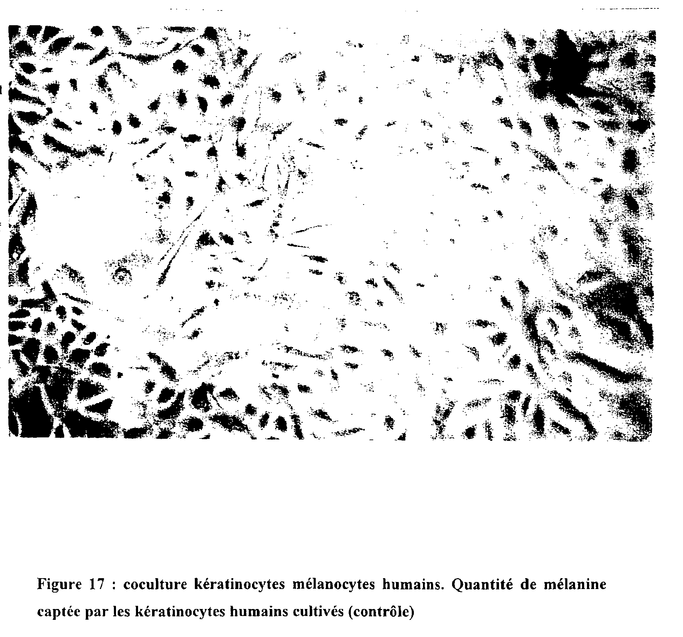

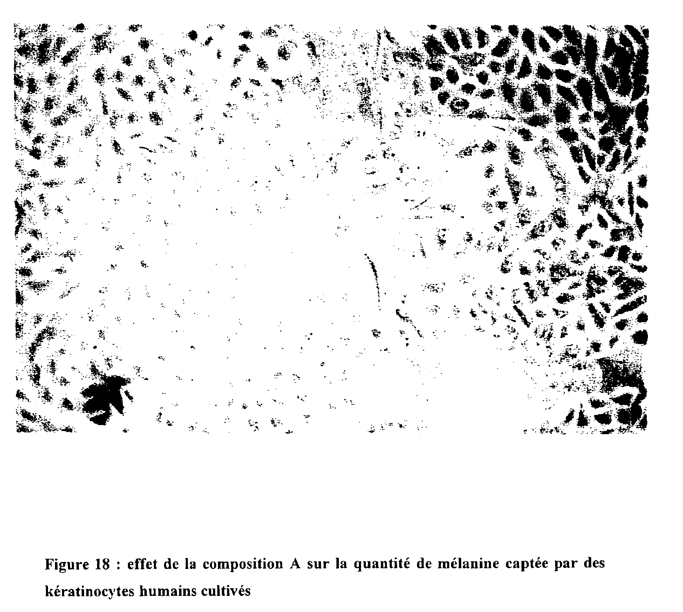

La présence de 1% de la composition A provoque un changement de répartition significatif de la mélanine captée par les kératinocytes en évitant les concentrations ponctuelles (voir les Figures 17 et 18).

### 1.4) Mise en évidence par immunocytochimie de la stimulation des kératinoctes et des fibroblastes par la composition A

### 1.4.1) Méthode

Il s'agit une technique semi-quantitative rapide qui permet d'apprécier le taux de chacune des protéines (cytokératine et élastine) présentes dans le cytoplasme cellulaire. 4.10⁴ cellules sont ensemencées par chambre de 0.9 cm² (lame de 8 chambres, NUNC) et mises en culture pendant une nuit. Après rinçage avec du tampon HBSS, le milieu à 1% de la composition A ou le milieu de contrôle (à 0 % de composition A) est ajouté (450 µl/chambre). Les cultures sont incubées 48 heures. Après élimination des surnageants et rinçage des cultures, les cellules sont fixées avec du paraformaldéhyde pendant 30 minutes à 4°C puis rincées avec du tampon PBS. 200µl d'anticorps anti-cytokératine et/ou anti-élastine sont ajoutés. L'incubation dure 30 minutes à température ambiante. Les surnageants sont alors éliminés et les cellules sont rincées avec le PBS. 200µl du deuxième anticorps conjugué à la fluorescéine sont additionnés. Après 30 minutes d'incubation à température ambiante, les surnageants sont éliminés et les cellules sont rincées au PBS. Les lames sont alors montées puis examinées au microscope à fluorescence inversée. Les quantités d'élastine ou de cytokératine synthétisées par les cellules sont proportionnelles à l'intensité de la fluorescence.

### 1.4.2) Résultats

L'addition de 1% de la composition A dans le milieu de culture des kératinocytes a pour effet d'augmenter la synthèse des cytokératines par les cellules kératinocytaires de l'épiderme. Cette stimulation a été observée d'une manière importante à partir de 0.5% de la composition A.

Lorsque les fibroblastes sont incubés in vitro en présence de la composition A (à 1 %) on assiste au bout de 48 heures à une augmentation très sensible de l'intensité de la fluorescence traduisant ainsi une stimulation de la synthèse de l'élastine par les fibroblastes du derme.

### 1.5) Activité anti-inflammatoire de la composition A : dosage de l'interleukine 1 (IL-1) sécrétée par les cellules promyélocytiques HL 60 traitées par la composition

### A

Au cours d'un processus d'inflammation, on assiste d'abord à une migration d'un grand nombre de cellules inflammatoires du sang périphérique vers les sites d'inflammation, puis à une sécrétion importante au niveau de ces sites de certaines cytokines et notamment de l'IL-1. Cette dernière est considérée comme ayant un rôle majeur.

Dans cette étude, la sécrétion de l'IL-1 est mesurée en utilisant une lignée cellulaire HL-60 en présence de la composition A.

La production de l'IL-1 est amplifiée par addition dans le milieu de culture de phytohémagglutinine (PHA) qui est un puissant stimulant de la sécrétion d'IL-1. L'IL-1 est quantifiée par la technique classique du dosage immunologique par ELISA à l'aide d'un anticorps spécifique dirigé contre l'IL-1 humain.

### 1.5.2) Résultats

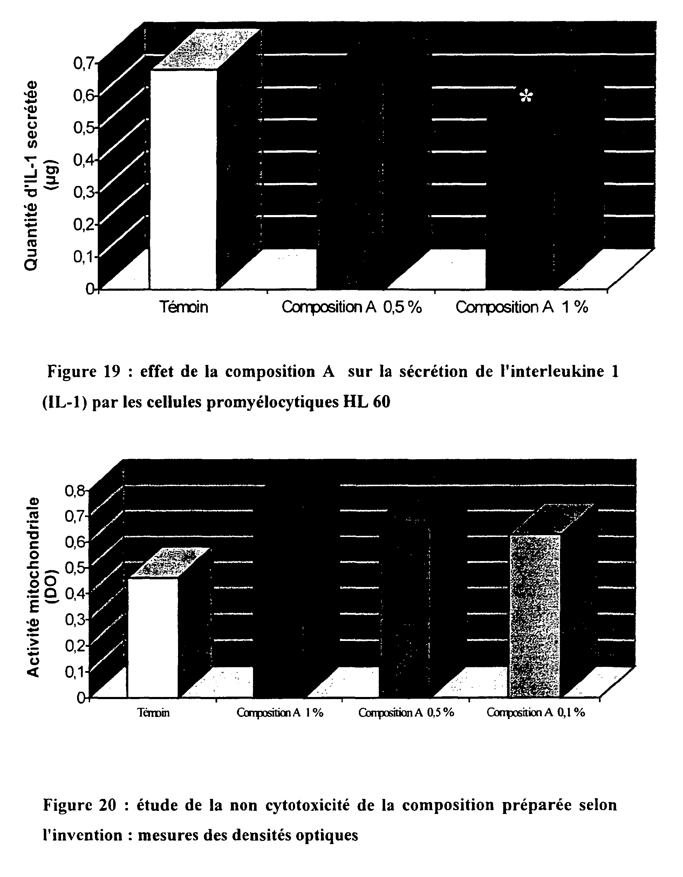

Les résultats sont regroupés dans le tableau 3 suivant et représentés par l'histogramme à la Figure 19.

**Tableau 3 : mise en évidence de l'effet de la composition A sur la production d'interleukine 1 (IL-1, en picogrammes) sécrétée par les cellules promyélocytiques**

| | **Echantillon 1** | **Echantillon 2** | **Echantillon 3** | **moyenne** | **écart type** |
|---|---|---|---|---|---|
| **Témoin** | **0,703** | **0,685** | **0,654** | **0,681** | **0,025** |
| **Composition A (0.5 %)** | **0,689** | **0,664** | **0,570** | **0,641** | **0,063** |
| **Composition A (1 %)** | **0,494** | **0,615** | **0,527** | **0,545** | **0,063** |

En présence de 1% de la composition A, on observe donc une baisse significative de la synthèse de l'IL-1 traduisant ainsi un effet anti-inflammatoire très sensible de la composition A. Cet effet in vitro a également été observé lors d'implantations pratiquées chez le rat in vivo.

### 1.6) Test de toxicité. Mise en évidence de la non cytotoxicité de la composition A : test M.T.T. d'activité mitochodriale

### 1.6.1) Méthode

Il s'agit d'un test colorimétrique basé sur la réduction d'un sel de tétrazolium (M.T.T.) par la NADPH réductase mitochondriale de cellules vivantes en un produit formazan bleu-violet.

2.10⁴ cellules dans 200 µl de milieu de culture sont ensemencées dans chaque puits (plaque 96, NUNC), puis incubées pendant une nuit pour leur permettre de bien adhérer au plastique. Après rinçage des cellules avec du milieu sans sérum, les différentes concentrations contenues dans le milieu de culture (1 %, 0.5 % et 0.1 % de composition A) ou le milieu témoin (0 % de composition A) sont ajoutées à valeur de 200 µl/puits.

Après 48 heures d'incubation, les surnageants sont éliminés et les cellules sont rincées avec du tampon HBSS. 100 µl de milieu de culture contenant 20 % d'une solution de M.T.T. (bromure de 3-4,5-diméthylthiazol-2-gamma,1-2,5-diphényltétrazolium; "Thiazolyl Blue") sont additionnés dans chaque puits. La réaction s'effectue pendant 2 heures à l'étuve à 37° C à 5 % de CO₂.

Pour permettre une meilleure solubilisation des cristaux de formazan, 100 µl de HCl 0.04N dans de l'isopropanol sont ajoutés puis la réaction colorée est lue à une longueur d'ondes de 570 nm par un lecteur de microplaques. Les valeurs sont également exprimées en densité optique.

### 1.6.2) Résultats

Les résultats sont regroupés dans le tableau 4 suivant et représentés par l'histogramme à la Figure 20.

**Tableau 4 : densités optique mesurées**

| | **Ech. 1** | **Ech. 2** | **Ech. 3** | **Ech. 4** | **Ech. 5** | **Ech.6** | **moyenne** | **écart type** |
|---|---|---|---|---|---|---|---|---|
| Témoin | 0,455 | 0,466 | 0,449 | 0,449 | 0,47 | 0,472 | 0,460 | 0,01 |
| Composition A (1%) | 0,732 | 0,796 | 0,771 | 0,785 | 0,808 | 0,768 | 0,777 | 0,027 |
| Composition A (0.5 %) | 0,669 | 0,675 | 0,709 | 0,7 | 0,698 | 0,694 | 0,691 | 0,016 |
| Composition A (0,1 %) | 0,643 | 0,648 | 0,617 | 0,624 | 0,621 | 0,628 | 0,630 | 0,013 |

La présence de la composition A dans le milieu de culture entraîne donc une augmentation importante de l'activité mitochondriale cellulaire. Cette stimulation de l'activité enzymatique est dose dépendante : plus la concentration en composition A est forte, plus l'activité est élevée. Mais, quelle que soit la teneur du milieu en composition A, l'activité mitochondriale des cellules est largement supérieure à celle des cellules témoins non traitées par la composition A.

### 2.7) Conclusions sur l'activité de la composition A évaluée in vitro

La composition A agit directement sur les couches les plus superficielles de l'épiderme.

Les composantes organiques et minérales de cet extrait s'expriment à plusieurs niveaux dans le métabolisme des kératinocytes en agissant sur l'activité des cellules de la couche basale.

Tout d'abord, la croissance, la division et la différenciation cellulaires sont stimulées puis les synthèses spécifiques des kératinocytes sont amplifiées, notamment celles des cytokératines et les médiateurs destinés au bon fonctionnement du derme comme par exemple le PTHrp favorisant la cascade de communications intercellulaires.

La séquence de différenciation des kératinocytes basaux, étoilés puis granulaires, est stimulée et régulée sans augmentation du nombre des cellules.

L'augmentation régulée du nombre des kératinocytes améliore la structure de l'épiderme et les jonctions dermo-épidermiques. La restructuration de l'épiderme contribue à une meilleure protection des couches les plus profondes.

L'amélioration des synthèses spécifiques telles que celles des cytokératines, stabilise les synthèses pigmentaires et assure une réparation physiologique de la mélanine au sein des kératinocytes se traduisant par l'absence de concentrations ponctuelles. L'amélioration de la protection cutanée se fait sans induire d'épaississement anormal de la peau, tout en contribuant à approfondir les plans cellulaires couchés sur la jonction dermo-épidermique.

C'est parmi l'ensemble des médiateurs sécrétés par les kératinocytes que l'on trouve des substances contribuant à améliorer la réponse de cellules à l'agression et à l'inflammation ainsi que les facteurs de communication permettant les échanges physiologiques entre épiderme et derme. Le renouvellement cellulaire de l'épiderme qui est de 4 à 6 semaines est maintenu à son rythme physiologique.

L'amplification de la synthèse des médiateurs augmente le recrutement de tout un ensemble de cellules et c'est parmi ces médiateurs que l'on trouve la Décorine apportée par la composition A au niveau épidermique.

### 2.7.1) Au niveau épidermique

En activant la synthèse de PTHrp, la composition A agit sur la croissance et la différenciation des kératinocytes. Cet effet est calcium dépendant, d'où l'intérêt de la présence du calcium ionisé dans la composition A.

L'induction de la division cellulaire et de l'index mitotique au niveau de la couche germinale se traduit par une réelle restructuration de l'épiderme qui devient plus résistant, plus profond et en continuelle interaction avec le derme qu'il protège de la déshydratation.

### 2.7.2) Au niveau dermique

La cascade de stimulation entraînée par les médiateurs locaux libérés au niveau de l'épiderme provoque la synthèse d'une très abondante matrice extracellulaire par les fibroblastes. Celle-ci est très structurée. Elle est notamment composée de protéoglycannes, de collagène et de protéines d'adhésion notamment de la Décorine, protéine d'adhésion jouant un rôle majeur dans les phénomènes de restructuration cutanée (ou régénération tissulaire cutanée). Elle piège les cytokines et autres facteurs de croissance. Ces observations ont également été constatées in vivo chez le rat.

Par ailleurs, l'enrichissement en élastine sous l'effet de la composition A dont les fibres sont mieux orientées dans la couche conjonctive, permet aux tissus cutanés de garder leur souplesse et leur forme au cours des dégradations physiologiques entraînées soit par les carences et l'âge, soit par les agressions notamment les étirements.

Les fibroblastes turgescents sont recrutés au niveau des cellules souches précurseurs en nombre suffisant pour que l'effet dû à leur action et leur présence soient durables. Cet effet est entretenu par une microvascularisation accrue.

La peau est plus élastique et plus ferme. Elle est résistante. Ses composantes se renouvellent à un rythme soutenu.

Au niveau des différents types cellulaires de la peau, la composition A présente une action réparatrice et régulatrice de l'équilibre physiologique entre ses différents constituants. De plus, il présente l'avantage de l'innocuité totale et celui d'avoir un effet anti-inflammatoire, donc apaisant.

### Exemple 3 : crème hydratante

| **Crème Hydratante** | |
|---|---|
| **Dénomination INCI** | **100 KG** |
| PEG-100 STEARATE GLYCERYL STEARATE | 1.000 |
| PARAFFINUM LIQUIDUM | 3.500 |
| LANOLINE | 1.700 |
| MYRISTYL ETOXY MYRISTATE | 4.000 |
| HUILES VEGETALES | 4.000 |
| CETYL ALCOHOL | 2.500 |
| TRIETHANOLAMINE | 0.200 |
| ANTIOXYDANT | 0.030 |
| PROPYL PARABEN | 0.300 |
| CARBOMER | 0.250 |
| DISODIUM EDTA | 0.400 |
| PROPYLENE GLYCOL | 2.000 |
| CONSERVATEUR | 0.600 |
| POLYACRYLAMIDE/C13-14 | 1.000 |
| ISOPARAFFIN/LAURETH-7 | |
| Composition A | 2.500 |
| PARFUM | TRACE |
| AQUA | QSP 100 |

## Revendications

1. Procédé de préparation d'une composition comprenant l'intégralité des composants de la nacre, **caractérisé en ce qu'**il comprend les étapes consistant :
a) à réduire de la nacre en une poudre de granulométrie comprise entre environ 1 et environ 300 µm;
b) à mettre la poudre de nacre ainsi obtenue en contact intime avec un agent d'extraction sous la forme d'une solution hydroglycolique d'au moins un collagène, d'au moins un protéoglycanne ou d'un mélange de ces derniers; puis
c) à récupérer le mélange d'extraction, formé par la mise en contact intime, constituant la composition désirée.

2. Procédé selon la revendication 1, **caractérisé en ce que** la nacre est réduite en une poudre de granulométrie comprise entre environ 50 et environ 100 µm.

3. Procédé selon la revendication 1, **caractérisé en ce que** la nacre est réduite en une poudre de granulométrie comprise entre environ 15 et environ 50 µm.

4. Procédé selon l'une quelconque des revendications précédentes, **caractérisé en ce que** le solvant hydroglycolique de l'agent d'extraction présente un rapport pondéral eau:glycol compris entre environ 1 : 100 et environ 100 : 1.

5. Procédé selon l'une quelconque des revendications précédentes, **caractérisé en ce que** l'agent d'extraction est une solution hydroglycolique d'au moins un collagène.

6. Procédé selon la revendication 5, **caractérisé en ce que** le collagène est un collagène marin.

7. Procédé selon la revendication 5 ou 6, **caractérisé en ce que** le collagène est un collagène marin choisi dans le groupe constitué par le "PANCOGENE^{R} MARIN", le "COLLAGENE NATIF MARIN - Code 690" et les mélanges de ces derniers.

8. Procédé selon l'une quelconque des revendications 5 à 7, **caractérisé en ce que** la concentration en collagène est comprise entre environ 0,0001 et environ 50 % en poids, par rapport au poids total de l'agent d'extraction.

9. Procédé selon l'une quelconque des revendications précédentes, **caractérisé en ce que** l'agent d'extraction est une solution hydroglycolique d'au moins un protéoglycanne.

10. Procédé selon la revendication 9, **caractérisé en ce que** le protéoglycanne est choisi dans le groupe constitué par l'acide hyaluronique, le condroitine sulfate, le dermatane sulfate, l'éparane sulfate, le kératane sulfate et les mélanges de ces derniers.

11. Procédé selon la revendication 9 ou 10, **caractérisé en ce que** l'agent d'extraction est une solution hydroglycolique d'acide hyaluronique.

12. Procédé selon l'une quelconque des revendications 9 à 11, **caractérisé en ce que** la concentration en protéoglycanne est de préférence comprise entre environ 0,0001 et environ 40 % en poids, par rapport au poids total de l'agent d'extraction.

13. Procédé selon l'une quelconque des revendications précédentes, **caractérisé en ce que** l'on met la poudre de nacre en contact intime avec l'agent d'extraction selon l'étape b) en réalisant un mélange, constitué par la poudre de nacre et l'agent d'extraction, tel qu'il comprend, par rapport à son poids total, environ 20 à environ 60 % en poids de poudre de nacre obtenue à l'étape a) et le reste en agent d'extraction.

14. Procédé selon l'une quelconque des revendications précédentes, **caractérisé en ce qu'**on réalise la mise en contact intime à l'étape b), pour une température donnée, pendant un temps suffisant pour réaliser une extraction pratiquement complète.

15. Procédé selon l'une quelconque des revendications précédentes, **caractérisé en ce que**, à la fin de l'étape b) on récupère le mélange d'extraction, formé par la mise en contact intime, constituant la composition désirée, et l'on sépare la phase liquide de la phase solide de la composition.

16. Composition comprenant l'intégralité des composants de la nacre susceptible d'être obtenue par le procédé selon l'une quelconque des revendications 1 à 15.

17. Composition selon la revendication 16, **caractérisée par le fait qu'**elle comprend au moins, sous forme d'une suspension hydroglycolique :
- de l'aragonite (CaCO₃);
- des oligo-éléments choisis dans le groupe constitué par le sodium, le magnésium, le lanthane, le zinc, le brome, le césium, le fer, le manganèse, le chlore, le cuivre, le potassium, le calcium, le strontium, le soufre et les mélanges de ces derniers;
- des protéines fibreuses de la nacre;
- des protéines non fibreuses de la nacre, et
- au moins un collagène non issu de la nacre et /ou au moins un protéoglycanne non issu de la nacre.

18. Composition selon la revendication 16 ou 17, **caractérisée en ce qu'**elle comprend au moins un collagène marin non issu de la nacre et au moins un protéoglycanne non issu de la nacre choisi dans le groupe constitué par l'acide hyaluronique, le condroitine sulfate, le dermatane sulfate, l'éparane sulfate, le kératane sulfate et les mélanges de ces derniers.

19. Composition pharmaceutique, **caractérisée en ce qu'**elle comprend la composition selon l'une quelconque des revendications 16 à 18, en tant que principe actif, et au moins un un excipient pharmaceutiquement acceptable.

20. Composition pharmaceutique selon la revendication 19, **caractérisée en ce** l'excipient pharmaceutiquement acceptable est un excipient approprié pour une application dermatologique.

21. Utilisation de la composition selon l'une quelconque des revendications 16 à 18, pour la fabrication d'un médicament destiné au traitement des troubles de la régénération tissulaire de la peau et/ou des phanères.

22. Utilisation de la composition selon l'une quelconque des revendications 16 à 18, pour la fabrication d'un médicament destiné au traitement des troubles de la peau et/ou des phanères liés au vieillissement.

23. Utilisation de la composition selon l'une quelconque des revendications 16 à 18, pour la fabrication d'un médicament destiné au traitement des manifestations cutanées inflammatoires.

24. Composition cosmétique, **caractérisée en ce qu'**elle comprend la composition selon l'une quelconque des revendications 16 à 18, en tant que principe cosmétiquement actif, et un excipient cosmétiquement acceptable.

25. Utilisation de la composition selon l'une quelconque des revendications 16 à 18, dans une composition cosmétique en tant qu'agent pour la régénération tissulaire de la peau et/ou des phanères.

26. Utilisation de la composition selon l'une quelconque des revendications 16 à 18, dans une composition cosmétique en tant qu'agent permettant de prévenir et/ou de réduire les effets liées au vieillissement de la peau et/ou des phanères.

27. Méthode de traitement cosmétique pour la régénération tissulaire de la peau et/ou des phanères, **caractérisée en ce qu'**on applique sur la peau et/ou les phanères la composition selon l'une quelconque des revendications 16 à 18.

28. Méthode de traitement cosmétique des modifications liées au vieillissement de la peau et/ou des phanères, **caractérisée en ce qu'**on applique sur la peau et/ou les phanères la composition selon l'une quelconque des revendications 16 à 20.

## Claims

1. Method for preparing a composition comprising all the components of mother-of-pearl, **characterized in that** it comprises the steps consisting:
a) in reducing mother-of-pearl to a powder with a particle size of between approximately 1 and approximately 300 µm;
b) in bringing the mother-of-pearl powder thus obtained into close contact with an extracting agent in the form of an aqueous-glycolic solution of at least one collagen, or of at least one proteoglycan or of a mixture thereof; and then
c) in recovering the extraction mixture formed as a result of the bringing into close contact, constituting the desired composition.

2. Method according to Claim 1, **characterized in that** the mother-of-pearl is reduced to a powder with a particle size of between approximately 50 and approximately 100 µm.

3. Method according to Claim 1, **characterized in that** the mother-of-pearl is reduced to a powder with a particle size of between approximately 15 and approximately 50 µm.

4. Method according to any one of the preceding claims, **characterized in that** the aqueous-glycolic solvent of the extracting agent has a water:glycol weight ratio of between approximately 1:100 and approximately 100:1.

5. Method according to any one of the preceding claims, **characterized in that** the extracting agent is an aqueous-glycolic solution of at least one collagen.

6. Method according to Claim 5, **characterized in that** the collagen is a marine collagen.

7. Method according to Claim 5 or 6, **characterized in that** the collagen is a marine collagen chosen from the group consisting of "PANCOGENE^{R} MARIN", "COLLAGENE NATIF MARIN - Code 690" and mixtures thereof.

8. Method according to any one of Claims 5 to 7, **characterized in that** the collagen concentration is between approximately 0.0001 and approximately 50% by weight, relative to the total weight of the extracting agent.

9. Method according to any one of the preceding claims, **characterized in that** the extracting agent is an aqueous-glycolic solution of at least one proteoglycan.

10. Method according to Claim 9, **characterized in that** the proteoglycan is chosen from the group consisting of hyaluronic acid, chondroitin sulphate, dermatan sulphate, heparan sulphate, keratan sulphate and mixtures thereof.

11. Method according to Claim 9 or 10, **characterized in that** the extracting agent is an aqueous-glycolic solution of hyaluronic acid.

12. Method according to any one of Claims 9 to 11, **characterized in that** the proteoglycan concentration is preferably between approximately 0.0001 and approximately 40% by weight, relative to the total weight of the extracting agent.

13. Method according to any one of the preceding claims, **characterized in that** the mother-of-pearl powder is brought into close contact with the extracting agent according to step b) by preparing a mixture, consisting of the mother-of-pearl powder and the extracting agent, such that it comprises, relative to its total weight, approximately 20 to approximately 60% by weight of mother-of-pearl powder obtained in step a) and the remainder as extracting agent.

14. Method according to any one of the preceding claims, **characterized in that** the close contact in step b) is brought about, for a given temperature, for a period of time sufficient to produce a virtually complete extraction.

15. Method according to any one of the preceding claims, **characterized in that**, at the end of step b), the extraction mixture, formed as a result of the bringing into close contact, which constitutes the desired composition, is recovered and the liquid phase of the composition is separated from the solid phase.

16. Composition comprising all the components of mother-of-pearl which can be obtained using the method according to any one of Claims 1 to 15.

17. Composition according to claim 16, **characterized in that** it comprises at least, in the form of an aqueous-glycolic suspension:
- aragonite (CaCO₃);
- trace elements chosen-from the group consisting of sodium, magnesium, lanthanum, zinc, bromine, caesium, iron, manganese, chlorine, copper, potassium, calcium, strontium, sulphur and mixtures thereof;
- fibrous proteins from mother-of-pearl;
- nonfibrous proteins from mother-of-pearl; and
- at least one collagen not derived from mother-of-pearl and/or at least one proteoglycan not derived from mother-of-pearl.

18. Composition according to Claim 16 or 17, **characterized in that** it comprises at least one marine collagen not derived from mother-of-pearl and at least one proteoglycan not derived from mother-of-pearl chosen from the group consisting of hyaluronic acid, chondroitin sulphate, dermatan sulphate, heparan sulphate, keratan sulphate and mixtures thereof.

19. Pharmaceutical composition, **characterized in that** it comprises the composition according to any one of Claims 16 to 18, as active principle, and at least one pharmaceutically acceptable excipient.

20. Pharmaceutical composition according to Claim 19, **characterized in that** the pharmaceutically acceptable excipient is an excipient suitable for dermatological application.

21. Use of the composition according to any one of Claims 16 to 18, for producing a medicinal product intended for the treatment of tissue regeneration disorders of the skin and/or superficial body growths.

22. Use of the composition according to any one of Claims 16 to 18, for producing a medicinal product intended for the treatment of disorders of the skin and/or superficial body growths related to aging.

23. Use of the composition according to any one of Claims 16 to 18, for producing a medicinal product intended for the treatment of inflammatory skin manifestations.

24. Cosmetic composition, **characterized in that** it comprises the composition according to any one of Claims 16 to 18, as cosmetically active principle, and a cosmetically acceptable excipient.

25. Use of the composition according to any one of Claims 16 to 18, in a cosmetic composition as an agent for tissue regeneration of the skin and/or superficial body growths.

26. Use of the composition according to any one of Claims 16 to 18, in a cosmetic composition as an agent making it possible to prevent and/or reduce the effects related to aging of the skin and/or superficial body growths.

27. Method of cosmetic treatment for tissue regeneration of the skin and/or superficial body growths, **characterized in that** the composition according to any one of Claims 16 to 18 is applied to the skin and/or superficial body growths.

28. Method of cosmetic treatment of modifications related to aging of the skin and/or superficial body growths, **characterized in that** the composition according to any one of Claims 16 to 20 is applied to the skin and/or superficial body growths.

## Patentansprüche

1. Verfahren zur Herstellung einer Zusammensetzung, welche die Gesamtheit der Bestandteile von Perlmutt umfasst, **dadurch gekennzeichnet, dass** es die Schritte umfasst, welche darin bestehen:
a) Perlmutt zu einem Pulver mit einer Granulometrie zwischen ungefähr 1 und ungefähr 300 µm eingeschlossen zu zerkleinern;
b) das so erhaltene Perlmuttpulver in innigen Kontakt mit einem Extraktionsmittel in Form einer wässrig-glycolischen Lösung von wenigstens einem Kollagen, wenigstens einem Proteoglycan oder einer Mischung von diesen Letzteren zu bringen; dann
c) die durch das innige Inkontaktbringen gebildete Extraktionsmischung, welche die gewünschte Zusammensetzung bildet, zu gewinnen.

2. Verfahren nach Anspruch 1, **dadurch gekennzeichnet, dass** das Perlmutt zu einem Pulver mit einer Granulometrie zwischen ungefähr 50 und ungefähr 100 µm eingeschlossen zerkleinert wird.

3. Verfahren nach Anspruch 1, **dadurch gekennzeichnet, dass** das Perlmutt zu einem Pulver mit einer Granulometrie zwischen ungefähr 15 und ungefähr 50 µm eingeschlossen zerkleinert wird.

4. Verfahren nach einem der vorangegangenen Ansprüche, **dadurch gekennzeichnet, dass** das wässrig-glycolische Lösemittel des Extraktionsmittel ein Gewichtsverhältnis Wasser:Glycol zwischen ungefähr 1:100 und ungefähr 100:1 eingeschlossen aufweist.

5. Verfahren nach einem der vorangegangenen Ansprüche, **dadurch gekennzeichnet, dass** das Extraktionsmittel eine wässrig-glycolische Lösung von wenigstens einem Kollagen ist.

6. Verfahren nach Anspruch 5, **dadurch gekennzeichnet, dass** das Kollagen ein marines Kollagen ist.

7. Verfahren nach Anspruch 5 oder 6, **dadurch gekennzeichnet, dass** das Kollagen ein marines Kollagen ist, welches ausgewählt wird aus der Gruppe, welche aus "PANCOGENE^{R} MARIN", "COLLAGENE NATIF MARIN - Code 690" und den Mischungen von diesen Letzteren gebildet wird.

8. Verfahren nach einem der Ansprüche 5 bis 7, **dadurch gekennzeichnet, dass** die Kollagen-Konzentration zwischen ungefähr 0,0001 und ungefähr 50 Gew.-% eingeschlossen bezogen auf das Gesamtgewicht des Extraktionsmittels liegt.

9. Verfahren nach einem der vorangegangenen Ansprüche, **dadurch gekennzeichnet, dass** das Extraktionsmittel eine wässrig-glycolische Lösung von wenigstens einem Proteoglycan ist.

10. Verfahren nach Anspruch 9, **dadurch gekennzeichnet, dass** das Proteoglycan ausgewählt wird aus der Gruppe, welche aus Hyaluronsäure, Chondroitinsulfat, Dermatansulfat, Eparansulfat, Keratansulfat und den Mischungen von diesen Letzteren gebildet wird.

11. Verfahren nach Anspruch 9 oder 10, **dadurch gekennzeichnet, dass** das Extraktionsmittel eine wässrig-glycolische Lösung von Hyaluronsäure ist.

12. Verfahren nach einem der Ansprüche 9 bis 11, **dadurch gekennzeichnet, dass** die Proteoglycan-Konzentration bevorzugt zwischen ungefähr 0,0001 und ungefähr 40 Gew.-% eingeschlossen bezogen auf das Gesamtgewicht des Extraktionsmittels liegt.

13. Verfahren nach einem der vorangegangenen Ansprüche, **dadurch gekennzeichnet, dass** man das Perlmuttpulver in innigen Kontakt mit dem Extraktionsmittel gemäß Schritt b) bringt, indem man eine Mischung, welche aus dem Perlmuttpulver und dem Extraktionsmittel gebildet wird, herstellt derart, dass sie bezogen auf ihr Gesamtgewicht ungefähr 20 bis ungefähr 60 Gew.-% Perlmuttpulver, welches in Schritt a) erhalten worden ist, und den Rest Extraktionsmittel umfasst.

14. Verfahren nach einem der vorangegangenen Ansprüche, **dadurch gekennzeichnet, dass** man das innige Inkontaktbringen des Schritts b) bei einer gegebenen Temperatur während einer Zeitspanne, welche ausreicht, um eine praktisch vollständige Extraktion auszuführen, ausführt.

15. Verfahren nach einem der vorangegangenen Ansprüche, **dadurch gekennzeichnet, dass** man am Ende von Schritt b) die durch das innige Inkontaktbringen gebildete Extraktionsmischung, welche die gewünschte Zusammensetzung bildet, gewinnt und man die flüssige Phase von der festen Phase der Zusammensetzung abtrennt.

16. Zusammensetzung, welche die Gesamtheit der Bestandteile von Perlmutt umfasst, welche durch das Verfahren nach einem der Ansprüche 1 bis 15 erhalten werden kann.

17. Zusammensetzung nach Anspruch 16, **dadurch gekennzeichnet, dass** sie zumindest, in Form einer wässrig-glycolischen Suspension, umfasst
- Aragonit (CaCO₃);
- Spurenelemente, ausgewählt aus der Gruppe, welche aus Natrium, Magnesium, Lanthan, Zink, Brom, Cäsium, Eisen, Mangan, Chlor, Kupfer, Kalium, Calcium, Strontium, Schwefel und den Mischungen von diesen Letzteren gebildet wird;
- faserförmige Proteine des Perlmutts;
- nicht-faserförmige Proteine des Perlmutts und
- wenigstens ein Kollagen, welches nicht aus dem Perlmutt stammt, und/oder wenigstens ein Proteoglycan, welches nicht aus dem Perlmutt stammt.

18. Zusammensetzung nach Anspruch 16 oder 17, **dadurch gekennzeichnet, dass** sie wenigstens ein marines Kollagen, welches nicht aus dem Perlmutt stammt, und wenigstens ein Proteoglycan, welches nicht aus dem Perlmutt stammt, ausgewählt aus der Gruppe, welche aus Hyaluronsäure, Chondroitinsulfat, Dermatansulfat, Eparansulfat, Keratansulfat und den Mischungen von diesen Letzteren gebildet wird, umfasst.

19. Pharmazeutische Zusammensetzung, **dadurch gekennzeichnet, dass** sie die Zusammensetzung nach einem der Ansprüche 16 bis 18 als Wirkstoff und wenigstens einen pharmazeutisch annehmbaren Träger umfasst.

20. Pharmazeutische Zusammensetzung nach Anspruch 19, **dadurch gekennzeichnet, dass** der pharmazeutisch annehmbare Träger ein Träger ist, der für eine dermatologische Anwendung geeignet ist.

21. Verwendung der Zusammensetzung nach einem der Ansprüche 16 bis 18 für die Herstellung eines Arzneimittels, welches für die Behandlung von Störungen der Geweberegeneration der Haut und/oder der hornartigen Auswüchse aus der Haut (Haare und sonstige Behaarung, Nägel, Zähne...) bestimmt ist.

22. Verwendung der Zusammensetzung nach einem der Ansprüche 16 bis 18 für die Herstellung eines Arzneimittels, welches für die Behandlung von Störungen der Haut und/oder der hornartigen Auswüchse aus der Haut (Haare und sonstige Behaarung, Nägel, Zähne...), welche mit dem Altern verbunden sind, bestimmt ist.

23. Verwendung der Zusammensetzung nach einem der Ansprüche 16 bis 18 für die Herstellung eines Arzneimittels, welches für die Behandlung von entzündlichen Hauterscheinungen bestimmt ist.

24. Kosmetische Zusammensetzung, **dadurch gekennzeichnet, dass** sie die Zusammensetzung nach einem der Ansprüche 16 bis 18 als kosmetisch wirksamen Wirkstoff und einen kosmetisch annehmbaren Träger umfasst.

25. Verwendung der Zusammensetzung nach einem der Ansprüche 16 bis 18 in einer kosmetischen Zusammensetzung als Mittel für die Geweberegeneration der Haut und/oder der hornartigen Auswüchse aus der Haut (Haare und sonstige Behaarung, Nägel, Zähne...).

26. Verwendung der Zusammensetzung nach einem der Ansprüche 16 bis 18 in einer kosmetischen Zusammensetzung als Mittel, welches erlaubt, die mit dem Altern der Haut und/oder der hornartigen Auswüchse aus der Haut (Haare und sonstige Behaarung, Nägel, Zähne...) verbundenen Wirkungen zu verhüten und/oder zu verringern.

27. Verfahren zur kosmetischen Behandlung für die Geweberegeneration der Haut und/oder der hornartigen Auswüchse aus der Haut (Haare und sonstige Behaarung, Nägel, Zähne...), **dadurch gekennzeichnet, dass** man auf die Haut und/oder die hornartigen Auswüchse aus der Haut (Haare und sonstige Behaarung, Nägel, Zähne...) die Zusammensetzung nach einem der Ansprüche 16 bis 18 aufträgt.

28. Verfahren zur kosmetischen Behandlung der mit dem Altern der Haut und/oder der hornartigen Auswüchse aus der Haut (Haare und sonstige Behaarung, Nägel, Zähne...) verbundenen Modifikationen, **dadurch gekennzeichnet, dass** man auf die Haut und/oder die hornartigen Auswüchse aus der Haut (Haare und sonstige Behaarung, Nägel, Zähne...) die Zusammensetzung nach einem der Ansprüche 16 bis 20 aufträgt.
